# EUROPEAN PATENT SPECIFICATION

(11) **EP 4 312 824 B1**
(45) Date of publication and mention of the grant of the patent: **06.08.2025**
(21) Application number: 22712719.8
(22) Date of filing: 07.03.2022
(51) Int. Cl.: A61B 17/22

(54) **DUAL GUIDEWIRE SYSTEM FOR CALCIFIED VALVES**
DOPPELFÜHRUNGSDRAHTSYSTEM FÜR KALZIFIZIERTE VENTILE
SYSTÈME DE FIL-GUIDE DOUBLE POUR VALVES CALCIFIÉES

(30) Priority: 22.03.2021 US 202163164438 P
(43) Date of publication of application: 07.02.2024
(73) Proprietor: Edwards Lifesciences Corporation, Irvine, CA 92614-5688 (US)
(72) Inventor: TRIPATHY, Sakyasingh, Irvine, CA 92614 (US); CAO, Hengchu, Irvine, CA 92614 (US); NIA, Nima, V., Irvine, CA 92614 (US); DOMINICK, Douglas Thomas, Irvine, CA 92614 (US); SU, Bingquan, Irvine, CA 92614 (US); SUGIJOTO, Amanda Tjipta, Irvine, CA 92614 (US)
(74) Representative: Eisenführ Speiser
(86) International application number: PCT/US2022/019188
(87) International publication number: WO 2022/203850

(56) References cited:
- EP-A1- 2 760 351
- WO-A1-2012/040865
- WO-A2-2018/045156
- US-A1- 2016 206 424

## Description

### RELATED APPLICATION

This application claims priority to U.S. Provisional Patent Application Serial No. 63/164,438, filed on Mar 22, 2021 and entitled DUAL GUIDEWIRE SYSTEM FOR CALCIFIED VALVES.

### BACKGROUND

### Field

The present disclosure generally relates to the field of heart implant devices and implant techniques.

### Description of Related Art

Heart valve dysfunctions, such as valve calcification, can complicate operations to implant medical devices or perform other operations in the heart. Valve stenosis occurs when the heart's valves narrow. This narrowing prevents the valve from opening fully, which reduces or blocks blood flow. When the blood flow through the valve is reduced or blocked, the heart needs to work harder to pump blood. Eventually, this extra work limits the amount of blood it can pump, and this can cause symptoms as well as possibly weaken the heart muscle. US 2016/0206424 A1 discloses a system for mitral valve replacement comprising a cylindrical sheath having first and second guide wires with spiral ends.

### SUMMARY

The claimed invention is defined in independent claim 1 and relates to a device for advancing a primary guidewire past a heart valve, the device comprising: a primary guidewire configured to advance past a heart valve; a secondary guidewire configured to pull open a leaflet of the heart valve while the primary guidewire advances past the heart valve; and a catheter containing both the primary guidewire and the secondary guidewire. Preferred configurations of the claimed invention are defined in dependent claims 2 to 15.

Also described herein are related aspects, examples, embodiments and arrangements useful for understanding the claimed invention, and which do not necessarily constitute embodiments of the claimed invention. The subject-matter for which protection is sought is defined by the claims.

Described herein are one or more methods and/or devices to facilitate implanting of devices through a calcified or otherwise narrowed heart valve, using at least two guidewires.

One general aspect includes a device for advancing a primary guidewire past a heart valve. The device includes a primary guidewire configured to advance past a heart valve. The device also includes a secondary guidewire configured to pull open a leaflet of the heart valve while the primary guidewire advances past the heart valve. The primary guidewire may be independently actuatable from the secondary guidewire. The device can also include a catheter containing both the primary guidewire and the secondary guidewire.

Implementations of the device may include one or more of the following features. The secondary guidewire may include a leaflet anchor configured to removably couple with the leaflet of the heart valve. In an embodiment, the leaflet anchor may include a needle configured to pierce through a proximal surface of the leaflet, and a hook configured to attach to a distal surface of the leaflet, the distal surface opposite the proximal surface.

In one embodiment, the leaflet anchor may include a suction cup configured to couple with a proximal surface of the leaflet. In one embodiment, the leaflet anchor may include a screw. In some embodiments, the leaflet anchor may include a cone shape or pyramid shape having: a pointed end; and a base surface opposite the pointed end; where the pointed end is configured to pierce a proximal surface of the leaflet and the base surface is configured to abut against a distal surface of the leaflet.

In one implementation, the leaflet anchor may include: a first prong actuatable to a first configuration and a second configuration; and a second prong; where in the first configuration, the first prong is substantially parallel to the second prong, and in the second configuration the first prong is substantially perpendicular to the second prong.

The device may include a sheath configured to hold the first prong in the first configuration with the second prong in response to the sheath being in a first position. The sheath may be configured to release the first prong to the second configuration in response to the sheath being in a second position, the second position reached by pulling the sheath away from a distal end of the leaflet anchor. In one embodiment, the first prong is configured to bend towards a right angle in the second configuration.

In one embodiment, the leaflet anchor may include: a first prong actuatable to a first configuration and a second configuration; a second prong; and a joint joining the first prong and the second prong at a point away from distal ends of the first prong and the second prong. In the first configuration, the first prong presses against the second prong, and in the second configuration the first prong is separated from the second prong. In the first configuration, the first prong and the second prong may be configured to hold a portion of the leaflet between them. The device may include a sheath configured to press the first prong against the second prong in the first configuration.

One general aspect includes a method for advancing a primary guidewire past a calcified leaflet. The method may include advancing a catheter configured to surround the primary guidewire and a secondary guidewire. The method can also include attaching a leaflet anchor of the secondary guidewire to the calcified leaflet of a valve. The method can also include retracting the secondary guidewire attached to the calcified leaflet to open the valve. The method may further include advancing the primary guidewire through the open valve.

Implementations of the method may include one or more of the following features. The method may include detaching the secondary guidewire from the calcified leaflet, and utilizing the primary guidewire to perform a medical procedure. For example, the medical procedure can be a transcatheter aortic valve replacement procedure. Retracting the secondary guidewire may include retracting the secondary guidewire through a first lumen of the catheter and advancing the primary guidewire may include advancing the primary guidewire through a second lumen of the catheter.

Another general aspect includes a guidewire configured to removably attach to a leaflet via a leaflet anchor. The guidewire can include a first prong actuatable to a closed configuration and an open configuration. The guidewire can also include a second prong. The guidewire can also include a sheath configured to move from a first position that compresses the first prong against the second prong into the closed configuration, to a second position that releases the first prong from the second prong into the open configuration. In the open configuration, the first prong separates from the second prong and may form a hook, and in the closed configuration, the first prong may form a needle with the second prong.

Implementations of the guidewire may include one or more of the following features. When inserting the leaflet anchor through a proximal surface of a calcified leaflet, the leaflet anchor may be in the closed configuration. When the leaflet anchor hooks to a distal surface of the calcified leaflet, the leaflet anchor may be in the open configuration.

### BRIEF DESCRIPTION OF THE DRAWINGS

Various embodiments are depicted in the accompanying drawings for illustrative purposes, and should in no way be interpreted as limiting the scope of the inventions. In addition, various features of different disclosed embodiments can be combined to form additional embodiments, which are part of this disclosure. Throughout the drawings, reference numbers may be reused to indicate correspondence between reference elements.
Figure 1 provides a cross-sectional view of a human heart.

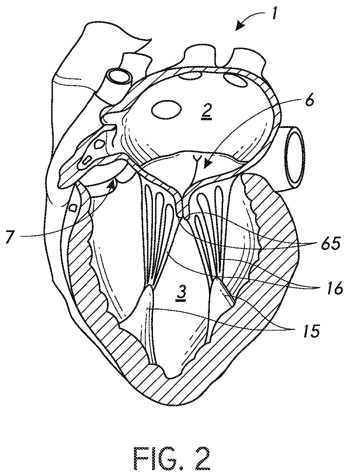

Figure 2 provides a cross-sectional view of the left ventricle and left atrium of an example heart.
Figures 3A and 3B illustrate a profile perspective of a dual guidewire device placed in the aortic valve according to certain embodiments.
Figures 4A and 4B illustrate a dual guidewire device having a hooking device for a leaflet anchor, according to certain embodiments.
Figures 5A and 5B illustrate a dual guidewire device having a suction device for a leaflet anchor, according to certain embodiments.
Figures 6A - 6C illustrate a secondary guidewire having an actuatable leaflet anchor and a sheath, according to certain embodiments.
Figure 7 provides a flow diagram representing a process for using the dual guidewire device 100 to perform a medical procedure, according to certain embodiments.

### DETAILED DESCRIPTION

Although certain preferred embodiments and examples are disclosed below, inventive subject matter extends beyond the specifically disclosed embodiments to other alternative embodiments and/or uses and to modifications and equivalents thereof. Thus, the scope of the claims that may arise herefrom is not limited by any of the particular embodiments described below. For example, with respect to any method or process disclosed herein, the acts or operations of the method or process may be performed in any suitable sequence and are not necessarily limited to any particular disclosed sequence.

Various operations may be described as multiple discrete operations in turn, in a manner that may be helpful in understanding certain embodiments; however, the order of description should not be construed to imply that these operations are order dependent. Additionally, the structures, systems, and/or apparatuses/devices described herein may be embodied as integrated components or as separate components. For purposes of comparing various embodiments, certain aspects and advantages of these embodiments are described. Furthermore, the headings provided herein are for convenience only and do not necessarily affect the scope or meaning of the claimed invention.

Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein. Similar reference numbers may be used with respect to separate diagrams and/or embodiments; use of such similar, or identical, reference numbers should not be interpreted as necessarily identifying identical components, and may refer to separate features.

### Overview

One cause of valve stenosis is the accumulation of deposits of calcium (valve calcification) on the heart valve. Calcium is a mineral found in the blood. As blood repeatedly flows over the valve, deposits of calcium can build up on the valve's cusps. These deposits may never cause any problems. However, in some people, particularly those with a congenitally abnormal valve, calcium deposits result in stiffening of the cusps of the valve. This stiffening narrows the valve. With calcified valves, getting a guidewire past the valve can take considerable time and expertise since the guidewire has to advance, typically against the blood flow, through a small opening due to the calcification.

The following disclosure discuses a dual guidewire system for guiding catheter insertion, where a primary guidewire is configured to guide a catheter to the target site in the heart while a secondary guidewire is configured to anchor itself to a calcified heart valve leaflet and hold open or otherwise enlarge the valve opening for the primary guidewire. The secondary guidewire can include a leaflet anchor for removably attaching to a valve leaflet. In one embodiment, the leaflet anchor includes a sharp needle with a hook, with the needle configured to pierce through a proximal side of the leaflet and the hook configured to attach against the distal side of the leaflet. Other embodiments of the attachment mechanism of the leaflet anchor can include adhesive, pinching arms, suction cups, and/or the like.

### Implantation Location

In humans and other vertebrate animals, the heart generally comprises a muscular organ having four pumping chambers, wherein the flow thereof is at least partially controlled by various heart valves, namely, the aortic, mitral (or bicuspid), tricuspid, and pulmonary valves. The valves may be configured to open and close in response to a pressure gradient present during various stages of the cardiac cycle (e.g., relaxation and contraction) to at least partially control the flow of blood to a respective region of the heart and/or to blood vessels (e.g., pulmonary, aorta, etc.).

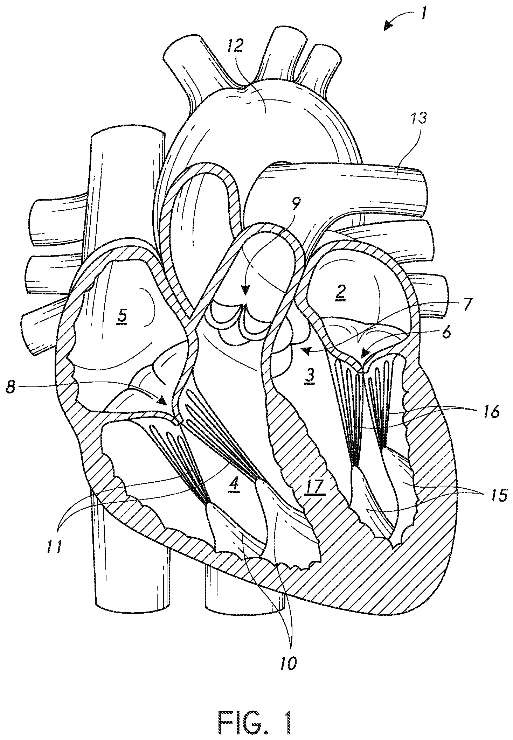

Figure 1 illustrates an example representation of a heart 1 having various features relevant to certain embodiments of the present inventive disclosure. The heart 1 includes four chambers, namely the left atrium 2, the left ventricle 3, the right ventricle 4, and the right atrium 5. A wall of muscle 17, referred to as the septum, separates the left 2 and right 5 atria and the left 3 and right 4 ventricles. The heart 1 further includes four valves for aiding the circulation of blood therein, including the tricuspid valve 8, which separates the right atrium 5 from the right ventricle 4. The tricuspid valve 8 may generally have three cusps or leaflets and may generally close during ventricular contraction (i.e., systole) and open during ventricular expansion (i.e., diastole). The valves of the heart 1 further include the pulmonary valve 9, which separates the right ventricle 4 from the pulmonary artery 13, and may be configured to open during systole so that blood may be pumped toward the lungs, and close during diastole to prevent blood from leaking back into the heart from the pulmonary artery. The pulmonary valve 9 generally has three cusps/leaflets, wherein each one may have a crescent-type shape. The heart 1 further includes the mitral valve 6, which generally has two cusps/leaflets and separates the left atrium 2 from the left ventricle 3. The mitral valve 6 may generally be configured to open during diastole so that blood in the left atrium 2 can flow into the left ventricle 3, and advantageously close during diastole to prevent blood from leaking back into the left atrium 2. The aortic valve 7 separates the left ventricle 3 from the aorta 12. The aortic valve 7 is configured to open during systole to allow blood leaving the left ventricle 3 to enter the aorta 12, and close during diastole to prevent blood from leaking back into the left ventricle 3.

Heart valves may generally comprise a relatively dense fibrous ring, referred to herein as the annulus, as well as a plurality of leaflets or cusps attached to the annulus. Generally, the size and position of the leaflets or cusps may be such that when the heart contracts, the resulting increased blood pressure produced within the corresponding heart chamber forces the leaflets at least partially open to allow flow from the heart chamber. As the pressure in the heart chamber subsides, the pressure in the subsequent chamber or blood vessel may become dominant, and press back against the leaflets. As a result, the leaflets/cusps come in apposition to each other such that the leaflets/cusps coapt, thereby closing the flow passage.

The atrioventricular (i.e., mitral 6 and tricuspid 8) heart valves may further comprise a respective collection of chordae tendineae (16, 11) and papillary muscles (15, 10) for securing the leaflets of the respective valves to promote and/or facilitate proper coaptation of the valve leaflets and prevent prolapse thereof. The papillary muscles (15, 10) may generally comprise finger-like projections from the ventricle wall, while the chordae tendineae (16, 11) may comprise cord-like tendons that connect the papillary muscles to the valve leaflets.

With respect to the mitral valve 6, a normal mitral valve may comprise two leaflets (anterior and posterior) and chordae tendineae 16 connecting the leaflets to two corresponding papillary muscles 15. The papillary muscles 15 originate in the left ventricle wall and project into the left ventricle 3. The valve leaflets of the mitral valve 6 may be prevented from prolapsing into the left atrium 2 by the action of the chordae tendineae 16 tendons connecting the valve leaflets to the papillary muscles 15. The relatively inelastic chordae tendineae 16 are attached at one end to the papillary muscles 15 and at the other to the valve leaflets; chordae tendineae from each of the papillary muscles 15 are attached to a respective leaflet of the mitral valve 6. Thus, when the left ventricle 3 contracts, the intraventricular pressure can force the valve to close, while the chordae tendineae 16 may keep the leaflets coapting together and prevent the valve from opening in the wrong direction, thereby preventing blood to flow back to the left atrium 2. The various cords of the chordae tendineae may have different thicknesses, wherein relatively thinner cords are attached to the free leaflet margin, while relatively thicker cords (e.g., strut cords) are attached farther away from the free margin.

With respect to the tricuspid valve 8, a normal tricuspid valve may comprise three leaflets (two shown in Figure 1) and three corresponding papillary muscles 10 (two shown in Figure 1). The leaflets of the tricuspid valve 8 may be referred to as the anterior, posterior and septal leaflets, respectively. The valve leaflets are connected to the papillary muscles by the chordae tendineae 11, which are disposed in the right ventricle 4 along with the papillary muscles 10. Although tricuspid valves are described herein as comprising three leaflets, it should be understood that tricuspid valves may occur with two or four leaflets in certain patients and/or conditions; the principles relating to papillary muscle binding and/or adjustment disclosed herein are applicable to atrioventricular valves having any number of leaflets and/or chordae tendineae or papillary muscles associated therewith. The right ventricular papillary muscles 10 originate in the right ventricle wall, and attach to the anterior, posterior and septal leaflets of the tricuspid valve, respectively, via the chordae tendineae 11. The papillary muscles 10 may serve to secure the leaflets of the tricuspid valve 8 to prevent prolapsing of the leaflets into the right atrium 5 during ventricular systole. Tricuspid regurgitation can be the result of papillary dysfunction or chordae rupture.

Heart valve disease represents a condition in which one or more of the valves of the heart fails to function properly. Diseased heart valves may be categorized as stenotic, wherein the valve does not open sufficiently to allow adequate forward flow of blood through the valve, and/or incompetent, wherein the valve does not close completely, causing excessive backward flow of blood through the valve when the valve is in a closed state. In certain conditions, valve disease can be severely debilitating and even fatal if left insufficiently treated. With regard to incompetent heart valves, over time and/or due to various physiological conditions, the position and/or tension of the chordae tendineae and/or papillary muscles may become altered, thereby pulling the valve leaflets at least partly open, which may cause valve regurgitation. For example, functional mitral valve regurgitation can occur when the left ventricle of the heart is distorted or dilated, displacing the papillary muscles, and chordae tendineae attached thereto, that support the mitral valve leaflets. For example, the valve leaflets may no longer come together to close the annulus, thereby resulting in blood flow back into the atrium. If left untreated, functional mitral valve regurgitation can overload the heart and can lead to or accelerate heart failure. Moving or pulling the chordae tendineae closer to the flow axis of the valve annulus according to their natural and healthy positions can potentially reduce occurrence of valve regurgitation.

Some types of valve disease result in leaflet calcification, which can complicate procedures that access the heart through a valve. Mitral annular calcification, characterized by calcium and lipid deposition in the annular fibrosa of the mitral valve, is a degenerative process commonly occurring in the elderly. Patients with mitral annular calcification may also have mitral valve leaflet calcification. In contrast to the mitral valve, calcification involving the tricuspid valve is rare. Isolated cases of tricuspid valve calcification have been reported associated with rheumatic heart disease, bacterial endocarditis, ventricular septal defect, and congenital malformations of the tricuspid valve.

Meanwhile, calcified stenosis of the aortic valve is a well-recognized and relatively common clinical and pathological entity. Aortic valve calcification is a condition in which calcium deposits form on the aortic valve in the heart. These deposits can cause narrowing at the opening of the aortic valve. This narrowing can become severe enough to reduce blood flow through the aortic valve. In contrast, calcific pulmonic stenosis is rarely encountered either at operation or necropsy. Some physicians have observed small deposits of calcium in one or more pulmonic valve cusps in patients who lived into adulthood with left-to right intracardiac shunts, and in older persons with severe pulmonary hypertension secondary to lung disease.

Various solutions disclosed herein relate to devices and methods for accessing the heart through calcified leaflets of a valve using a dual guidewire system. Such devices and methods can be used during procedures including transcatheter aortic valve replacement (TAVR) or transcatheter aortic valve implantation (TAVI). Usually valve replacement requires an open-heart procedure with a "sternotomy," in which the chest is surgically separated (opened) for the procedure. The TAVR or TAVI procedures can be done through very small openings that leave all the chest bones in place. For example, the TAVR procedure is performed using one of two approaches: entering through the femoral artery (large artery in the groin) using the transfemoral approach, which does not require a surgical incision in the chest; or using a minimally invasive surgical approach with a small incision in the chest and entering through a large artery in the chest or through the tip of the left ventricle (the apex) using a transapical approach.

### Dual Guidewire Device

As referenced above, certain embodiments disclosed herein provide for systems, devices, and methods for advancing a primary guidewire past a calcified leaflet while a secondary guidewire enlarges the valve aperture by pulling on the calcified leaflet. Such devices may be introduced into the patient system through surgical or, advantageously, minimally-invasive means.

A guidewire is a wire or spring typically used as a guide for placement of a larger device or prosthesis, such as a catheter or replacement valve. Guidewires are designed to navigate vessels to reach a target destination. Once the tip of the device arrives at its destination, it acts as a guide that larger catheters can rapidly follow for easier delivery to the treatment site. Guidewires can vary in size, length, stiffness, composition, and shape of the tip.

Generally, guidewires consist of four major components: a core, a wire tip, a body, and a coating. The inner part of the wire is called the core. Typically, the core is either made of very flexible nitinol or stiff stainless steel, which dictates a guidewire's flexibility. The tip refers to the distal end of the wire. Often, the end of the wire tip is wrapped in a ribbon of flexible metal to make the tips more flexible and atraumatic. The body of the wire, surrounding the core, is typically made of coils or polymers. Generally, the body of the wire (e.g., spring coil or polymer cover) is coated by an overlay, a specific material which gives the wire the ability to reduce surface friction, and improve device interaction and guidewire tracking. Depending on the intended use, the coating may be hydrophilic, hydrophobic, or have some other property to aid insertion.

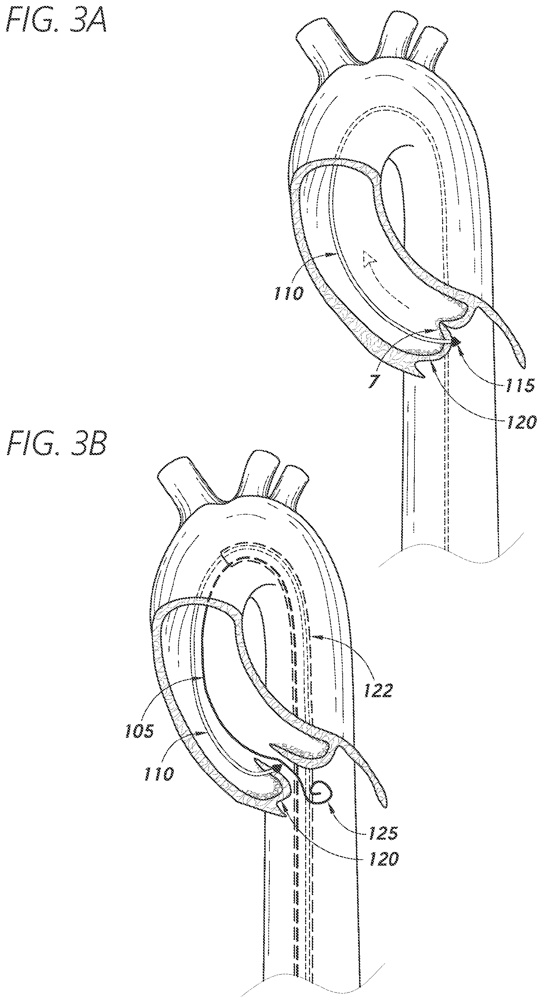

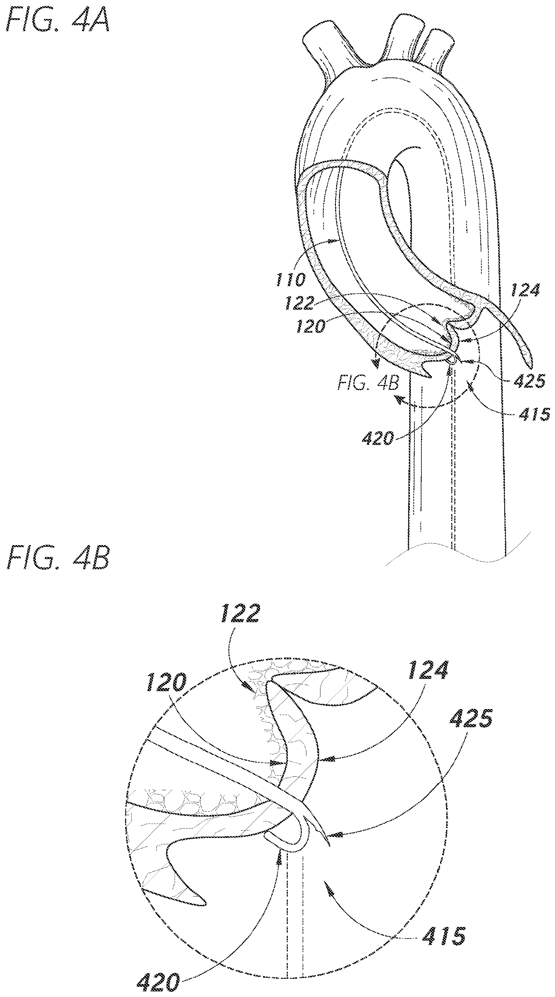

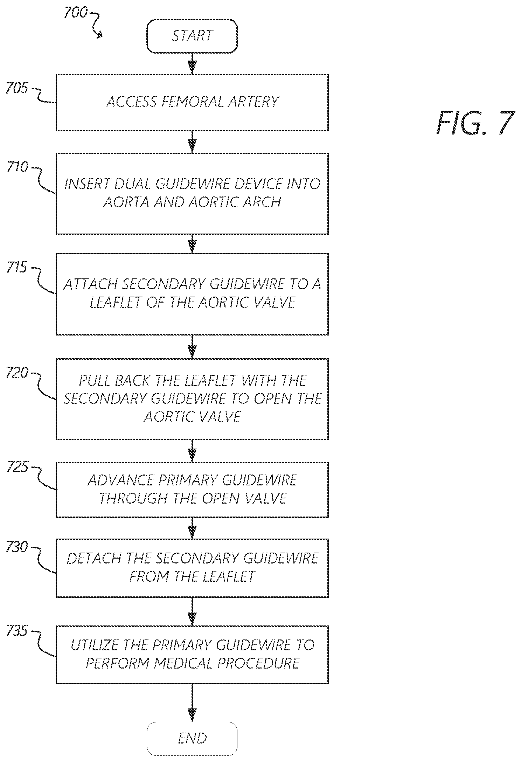

While each of Figures 3-7 may illustrate medical implants and/or processes including features for advancing the primary guidewire past a calcified leaflet using the secondary guidewire, these features may be used independently of each other or in combination with each other. For example, a dual guidewire device may use various types of leaflet anchors, as shown in Figures 4A-6C. Various embodiments of the dual guidewire device that utilize different leaflet anchors may use the process of Figure 7.

Figures 3A and 3B illustrates a profile perspective of a dual guidewire device 100 placed in the aortic valve 7, according to certain embodiments. The dual guidewire device 100 can comprise a primary guidewire 105, a secondary guidewire 110, and a catheter (not shown) to contain the primary and secondary guidewires and facilitate movement of the device through the body. The secondary guidewire 110 can include a leaflet anchor 115 for attaching to a leaflet 120 of the aortic valve. Various structures can be used as the leaflet anchor. For example, the leaflet anchor can be configured to pierce through the leaflet and anchor to the other side (distal surface). In other embodiments, the leaflet anchor is configured to attach to the facing side (proximal surface) of the leaflet.

In one example, the leaflet anchor is a pointed shape with a wider base (e.g., cone, pyramid, etc.) as shown in Figures 3A and 3B, where the pointed end can pierce through the proximal surface of the leaflet. After the leaflet anchor 115 passes through the leaflet 120, a distal surface of the base, opposite the pointed end, can abut against the distal surface of the leaflet, as shown in Figure 3A.

As shown in Figure 3B, the secondary guidewire 110 can be pulled, along with the attached leaflet 120, to enlarge the valve opening. When calcified, the leaflets typically are not pliable enough to fully open, providing a smaller opening for the primary guidewire 105 to pass through. To alleviate this, the secondary guidewire 110 can be pulled either manually or using a pulling mechanism (e.g., a reel, winch, or the like) that may be built into a handle of the device to widen the opening of the aortic valve 7. The distal end 125 of the primary guidewire can then be advanced past the aortic valve 7.

Figure 4A and Figure 4B illustrate a dual guidewire device 100 having a hooking device 415 for a leaflet anchor, according to certain embodiments. Figure 4B illustrates a close-up view of the guidewire device 100 of Figure 4A. The hooking device 415 can include a hook 420 and a needle 425. In an exemplary configuration, the end of the needle 425 forms the furthest distal point of the hooking device. Meanwhile, the hook 420 is formed proximally from the needle 425, with the interior of the arc formed by the hook 420 facing proximally and the exterior of the arc formed by the hook 420 facing distally relative to the shaft of the secondary guidewire 110. In some embodiments, the needle 425 is straight relative to the shaft of the secondary guidewire. However, in some embodiments the needle 425 may be slanted or curve towards the exterior arc of the hook 420. By bending the end of the needle 425 closer to the hook 420, the contact point (tip of the needle and top of exterior arc) with the leaflet of the hooking device 415 can be made narrower, thereby reducing the size of the tear made in the leaflet when pushing through the hooking device 415.

In an exemplary operation, the needle 425 can pierce through the leaflet 120 from the proximal surface 122 (towards the entry point of the guidewire device 100), creating an entry point through which the hook 420 can pass through the leaflet 120. The hook 420 can then engage with the distal surface 124 (away from the entry point of the guidewire device 100) of the leaflet 120.

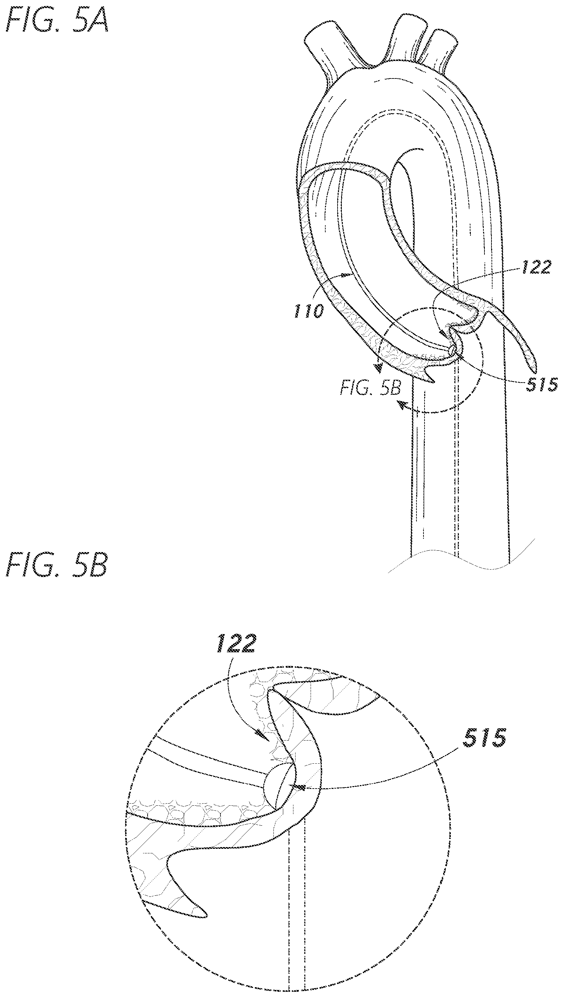

Figure 5A and Figure 5B illustrate a dual guidewire device 100 having a suction device 515 for a leaflet anchor, according to certain embodiments. Figure 5B illustrates a close-up view of the guidewire device 100 of Figure 5A. The suction device 515 can attach to the proximal surface 122 of the leaflet 120. The suction device 515 comprises a cup formed by a pliable material. When pushed against the leaflet, the cup compresses and expels the fluid within. As the cup attempts to reform to its original shape, the rim of the cup presses against the leaflet, forming a seal. With the cup sealed, the inside forms a vacuum, attaching the leaflet to the cup.

Because the suction device 515 attaches to the proximal surface 122, there may be less damage done to the leaflet 120 as piercing through is unnecessary. While some operations may be for implanting an artificial valve, other operations may leave the existing valve in place. In those situations, it may be beneficial to limit damage to the leaflets of the valve to make repair easier. Other embodiments of the dual guidewire device 100 can function similarly by attaching to the proximal surface, but use alternate embodiments of the leaflet anchor, such as screw devices or adhesive to attach to the leaflet 120.

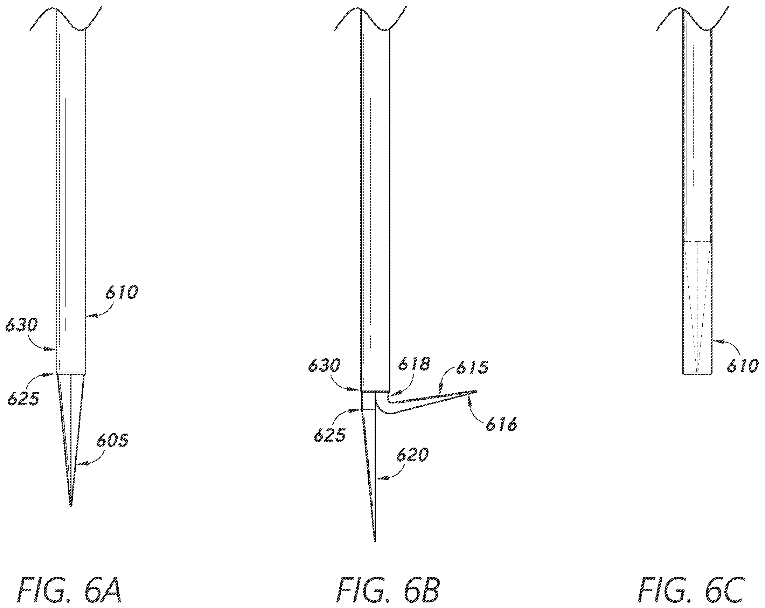

Figures 6A - 6C illustrate a secondary guidewire 110 having an actuatable leaflet anchor 605 and a sheath 610. In the illustrated embodiment, the actuatable leaflet anchor comprises two prongs 615, 620. The first prong 615 can be biased such that its end 616 bends into a substantially horizonal position relative to its shaft 618, forming a hook. For example, the prong material (e.g., spring steel) may be pliable but springs back to its original bent shape. The bent shape may form a right angle, but can differ by several degrees as long as the first prong can still hook against the leaflet. In some embodiments, movement of the sheath 610 over the actuatable leaflet anchor 605 causes the actuatable leaflet anchor to change configuration, as further discussed below.

In the first configuration shown in Figure 6A, the end of the sheath 610 is in a first location 625 relative to the actuatable leaflet anchor 605, preventing the biasing prong 615 from moving to the horizonal position. Instead, the biasing prong 615 and the second prong 620 are pressed together by the sheath to from a needle. Both prongs may be tapered to a point, such that the joined prongs together form the needle, as shown in Figure 6A. In the first configuration, the needle formed by the actuatable leaflet anchor 605 allows the actuatable leaflet anchor 605 to pierce through a leaflet of a valve more easily.

In the second configuration shown in Fig. 6B, the end of the sheath 610 moves to a second location 630 located distally to the first location 625, thereby exposing more of structure of the actuatable leaflet anchor 605. Movement from the first location 625 to the second location 630 may be accomplished, for example, by the user pulling on the sheath, manually or through a mechanism. By pulling the sheath to the second location 630, the biasing prong 615 is unbound by the sheath 610, allowing the biasing prong 615 to bend and form a hook structure.

In one exemplary use case, after pushing through the secondary guidewire 110 through a valve leaflet while in the first configuration, the user can pull the sheath 610 to the second location 630. The actuatable leaflet anchor 605 then changes from the first configuration to the second configuration. With the biasing prong 615 in the horizontal position, the biasing prong 615 abuts against the distal surface of the leaflet, thereby increasing the surface area against the leaflet. This allows greater force to be applied to the leaflet before the actuatable leaflet anchor 605 damages the leaflet, increasing the chance that the secondary guidewire 110 can pull open the leaflet to a position that enlarges the valve opening to the desired degree.

As discussed above, the actuatable leaflet anchor 605 can be made in a variety of forms, such as a needle, anchor, adhesive, pointed structure with a widened base, or the like. In addition, various types of material can be used for the leaflet anchor and the guidewires, such as nitinol, stainless steel, or other biocompatible material.

While the above has described using the actuatable leaflet anchor 605 to pierce through the leaflet 120 and attach to the distal surface 124 (shown in Figure 4B), the actuatable leaflet anchor 605 may also be used to attach to the proximal surface of the leaflet by pinching the leaflet between the two prongs 615, 620. In the open configuration shown in Figure 6B, the sheath 610 is at the second location 630 and the actuatable leaflet anchor 605 is open, with the first prong 615 and second prong 620 away from each other. The actuatable leaflet anchor 605 can be advanced towards the leaflet until the second prong 620 presses into the leaflet. The sheath 610 can then be moved to the first location 625, thereby pressing together the first prong 615 and the second prong 620, as shown in the closed configuration of Figure 6A. With the prongs 615, 620 in the closed position, a portion of the leaflet is pinched between the two prongs, attaching the actuatable leaflet anchor 605 to the leaflet. The actuatable leaflet anchor 605 can then be pulled to open the valve.

In the alternate usage described above, the actuatable leaflet anchor 605 may be adapted to better pinch the leaflet. For example, the first prong 615 and second prong 620 may have wider, flatter ends, to increase the contact surface area that pinches the leaflet tissue. In another example, the first prong 615 and second prong 620 may be attached together by a joint below the ends of the prongs, similar to pliers and scissors, to better facilitate the pinching motion by the two prongs. A spring or other biasing material may be located near the joint in order to push open the two prongs, unless held together by the sheath 610. In that implementation, the sheath 610 can be used to close the prongs together by advancing distally and release the prongs by retreating proximally.

### Guidewire Usage

Figure 7 provides a flow diagram representing a process for using the dual guidewire device 100 to perform a medical procedure, according to one or more embodiments disclosed herein. A health provider, such as a surgeon, can use the process during a transseptal, trans aorta, transfemoral artery, trans radial, transapical, or other surgical approach to perform a medical procedure (e.g., installing a stent, valve, or other implant) that requires access through a heart valve. For ease of explanation, the following describes a transfemoral approach to the aortic valve, such as used during a TAVR procedure. However, the process may be performed using other approaches and/or targeting other heart valves. In addition, for ease of explanation, the following uses the label numbering from previous figures. However, the process is not limited to those specific embodiments illustrated in those figures.

At block 705, the health provider gains access to the femoral artery from an insertion site on the patient's body. The health provider can insert a catheter at the insertion site and into the femoral artery. In some embodiments, the catheter may have separate lumens for the primary guidewire 105 and the secondary guidewire 110. Other embodiments may use a single lumen, with the guidewires sharing the lumen. In some embodiments, more than two guidewires may be used, with separate lumens for each guidewire.

At block 710, the health provider inserts the dual guidewire device 100 into the aorta and aortic arch, to approach the aortic valve. In some embodiments, one or more guidewires may already be in the catheter while the catheter is inserted into the patient's body. In other embodiments, the catheter is empty, with the guidewires inserted after the catheter reaches the target site (e.g., aortic valve) in the body.

At block 715, the health provider attaches the secondary guidewire to a leaflet of the aortic valve. As discussed above, various types of leaflet anchors can be used, from mechanical to adhesive, to attach to a calcified leaflet.

In some embodiments, the secondary guidewire may be attached to the proximal surface of the leaflet. For example, the secondary guidewire may use a suction cup or adhesive to attach to the surface. In one embodiment, the leaflet anchor is a screw that is screwed into the leaflet, for example, by rotating the guidewire to drive the screw into the leaflet tissue. In another embodiment, the leaflet anchor includes a pair of jaws or prongs that pivot on a joint, similar to pliers, that pinch the leaflet between the jaws or prongs.

In other embodiments, the secondary guidewire may pierce through the leaflet and press against the distal surface of the leaflet. For example, the leaflet anchor can be a needle and hook structure, a tapered cone or pyramid structure, or some of the embodiments described in the earlier figures.

At block 720, the health provider pulls back the leaflet with the secondary guidewire to open the aortic valve. In some embodiments, the dual guidewire device may have a winch, reel, or other mechanism to pull the guidewire attached to the end of the guidewire, outside the patient's body. In some embodiments, the health provider may manually pull on the secondary guidewire.

At block 725, the secondary guidewire pulls the calcified leaflet and widens the valve opening, allowing the primary guidewire to be pushed through the enlarged opening. In some embodiments, multiple guidewires may be used to attached to multiple leaflets. For example, if a single guidewire pulling on a single leaflet does sufficiently enlarge the opening, a second guidewire can be attached to a second leaflet to further open the valve. Even a third guidewire can be used to attached to a third leaflet (e.g., when opening a tricuspid valve).

At block 730, the secondary guidewire can be detached from the leaflet. For example, for leaflet anchors that pierce through the leaflet and abut against the distal surface of the leaflet, a covering sheath may be moved over the leaflet anchor, allowing the leaflet anchor to be pulled back through the leaflet. For leaflet anchors that attach to the proximal surface of the leaflet, various detachment methods can be used to detach the leaflet anchor. For example, a screw anchor can be unscrewed, a jaw anchor can be opened to release the leaflet, and a suction cup can be lifted to release a vacuum seal. In some embodiments, an adhesive anchor may use an adhesive that is formulated to dissolve after a certain amount of time, thereby releasing the leaflet. Once detached, the secondary guidewire can be pulled out of the catheter and out of the patient's body.

At block 735, the primary guidewire can then be guided to its destination in the heart, where it can be used in performing various possible medical procedures, such as a TAVR procedure or other procedure for implanting a device or operating on the heart.

### Additional Embodiments

Depending on the embodiment, certain acts, events, or functions of any of the processes described herein can be performed in a different sequence, may be added, merged, or left out altogether. Thus, in certain embodiments, not all described acts or events are necessary for the practice of the processes. Moreover, in certain embodiments, acts or events may be performed concurrently, rather than sequentially.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be understood that certain ordinal terms (e.g., "first" or "second", "primary" or "secondary") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," "proximal," "distal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

It should be appreciated that in the above description of embodiments, various features are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s). Further, no component, feature, step, or group of components, features, or steps are necessary or indispensable for each embodiment. Thus, it is intended that the scope of the inventions herein disclosed and claimed below should not be limited by the particular embodiments described above, but should be determined only by the claims that follow.

## Claims

1. A device (100) for advancing a primary guidewire (105) past a heart valve, the device (100) comprising:
a primary guidewire (105) configured to advance past a heart valve;
a secondary guidewire (110) configured to pull open a leaflet (120) of the heart valve while the primary guidewire (105) advances past the heart valve; and
a catheter containing both the primary guidewire (105) and the secondary guidewire (110).

2. The device (100) of claim 1, wherein the secondary guidewire (110) comprises a leaflet anchor (115) configured to removably couple with the leaflet (120) of the heart valve.

3. The device (100) of claim 2, wherein the leaflet anchor (115) comprises a needle (425) configured to pierce through a proximal surface of the leaflet (120), and a hook (420) configured to attach to a distal surface of the leaflet (120), the distal surface opposite the proximal surface.

4. The device (100) of claim 2, wherein the leaflet anchor (115) comprises a suction cup configured to couple with a proximal surface of the leaflet (120).

5. The device (100) of claim 2, wherein the leaflet anchor (115) comprises a screw.

6. The device (100) of claim 2, wherein the leaflet anchor (115) comprises a cone shape or pyramid shape having a pointed end and a base surface opposite the pointed end, wherein the pointed end is configured to pierce a proximal surface of the leaflet (120) and the base surface is configured to abut against a distal surface of the leaflet (120).

7. The device (100) of claim 2, wherein the leaflet anchor (115) comprises a first prong (615) actuatable to a first configuration and a second configuration, and a second prong (620), wherein in the first configuration the first prong (615) is substantially parallel to the second prong (620), and in the second configuration the first prong (615) is substantially perpendicular to the second prong (620).

8. The device (100) of claim 7, and further comprising a sheath (610) configured to hold the first prong (615) in the first configuration with the second prong (620) in response to the sheath (610) being in a first position.

9. The device (100) of claim 8, wherein the sheath (610) is configured to release the first prong (615) to the second configuration in response to the sheath (610) being in a second position, the second position reached by pulling the sheath (610) away from a distal end of the leaflet anchor (115).

10. The device (100) of any of claims 7-9, wherein the first prong (615) is configured to bend towards a right angle in the second configuration.

11. The device (100) of claim 2, wherein the leaflet anchor (115) comprises:
a first prong (615) actuatable to a first configuration and a second configuration;
a second prong (620); and
a joint joining the first prong (615) and the second prong (620) at a point away from distal ends of the first prong (615) and the second prong (620);
wherein in the first configuration, the first prong (615) presses against the second prong (620), and in the second configuration the first prong (615) is separated from the second prong (620).

12. The device (100) of claim 11, wherein in the first configuration the first prong (615) and the second prong (620) are configured to hold a portion of the leaflet (120) between them.

13. The device (100) of claim 12, and further comprising a sheath (610) configured to press the first prong (615) against the second prong (620) in the first configuration.

14. The device (100) of claim 2, wherein the leaflet anchor (115) comprises an adhesive, preferably an adhesive that is formulated to dissolve after a certain amount of time.

15. The device (100) of any of claims 1-14, wherein the primary guidewire (105) is independently actuatable from the secondary guidewire (110).

## Patentansprüche

1. Vorrichtung (100) zum Vorschieben eines Primärführungsdrahtes (105) über eine Herzklappe hinweg, wobei die Vorrichtung (100) umfasst:
einen Primärführungsdraht (105), der dazu konfiguriert ist, über eine Herzklappe hinweg vorgeschoben zu werden;
einen Sekundärführungsdraht (110), der dazu konfiguriert ist, ein Segel (120) der Herzklappe aufzuziehen, während der Primärführungsdraht (105) über die Herzklappe hinweg vorgeschoben wird; und
einen Katheter, der sowohl den Primärdraht (105) als auch den Sekundärdraht (110) enthält.

2. Vorrichtung (100) gemäß Anspruch 1, wobei der Sekundärführungsdraht (110) einen Segelanker (115) umfasst, der dazu konfiguriert ist, sich lösbar mit dem Segel (120) der Herzklappe zu koppeln.

3. Vorrichtung (100) gemäß Anspruch 2, wobei der Segelanker (115) eine Nadel (425) umfasst, die dazu konfiguriert ist, eine proximale Oberfläche des Segels (120) zu durchdringen, und einen Haken (420), der dazu konfiguriert ist, sich an einer distalen Oberfläche des Segels (120) zu befestigen, wobei die distale Oberfläche der proximalen Oberfläche gegenüberliegt.

4. Vorrichtung (100) gemäß Anspruch 2, wobei der Segelanker (115) einen Saugnapf umfasst, der dazu konfiguriert ist, sich mit einer proximalen Oberfläche des Segels (120) zu koppeln.

5. Vorrichtung (100) gemäß Anspruch 2, wobei der Segelanker (115) eine Schraube umfasst.

6. Vorrichtung (100) gemäß Anspruch 2, wobei der Segelanker (115) eine Kegelform oder Pyramidenform aufweist, mit einem spitzen Ende und einer der Spitze gegenüberliegenden Grundfläche, wobei das spitze Ende dazu konfiguriert ist, eine proximale Oberfläche des Segels (120) zu durchdringen, und die Grundfläche dazu konfiguriert ist, an einer distalen Oberfläche des Segels (120) anzuliegen.

7. Vorrichtung (100) gemäß Anspruch 2, wobei der Segelanker (115) einen ersten Zacken (615) umfasst, der in eine erste und eine zweite Konfiguration steuerbar ist, und einen zweiten Zacken (620), wobei in der ersten Konfiguration der erste Zacken (615) im Wesentlichen parallel zum zweiten Zacken (620) ist, und in der zweiten Konfiguration der erste Zacken (615) im Wesentlichen senkrecht zum zweiten Zacken (620) ist.

8. Vorrichtung (100) gemäß Anspruch 7, ferner umfassend eine Hülle (610), die dazu konfiguriert ist, den ersten Zacken (615) in der ersten Konfiguration mit dem zweiten Zacken (620) zu halten, als Reaktion darauf, dass sich die Hülle (610) in einer ersten Position befindet.

9. Vorrichtung (100) gemäß Anspruch 8, wobei die Hülle (610) dazu konfiguriert ist, den ersten Zacken (615) in die zweite Konfiguration freizugeben, als Reaktion darauf, dass sich die Hülle (610) in einer zweiten Position befindet, wobei die zweite Position erreicht wird durch Wegziehen der Hülle (610) vom distalen Ende des Segelankers (115).

10. Vorrichtung (100) gemäß einem der Ansprüche 7 bis 9, wobei der erste Zacken (615) dazu konfiguriert ist, sich in der zweiten Konfiguration zu einem rechten Winkel zu biegen.

11. Vorrichtung (100) gemäß Anspruch 2, wobei der Segelanker (115) umfasst:
einen ersten Zacken (615), der in eine erste Konfiguration und eine zweite Konfiguration steuerbar ist;
einen zweiten Zacken (620); und
ein Gelenk, das den ersten Zacken (615) und den zweiten Zacken (620) an einem Punkt weg von distalen Enden des ersten Zackens (615) und des zweiten Zackens (620) verbindet;
wobei in der ersten Konfiguration, der erste Zacken (615) gegen den zweiten Zacken (620) drückt, und in der zweiten Konfiguration der erste Zacken (615) vom zweiten Zacken (620) getrennt ist.

12. Vorrichtung (100) gemäß Anspruch 11, wobei in der ersten Konfiguration der erste Zacken (615) und der zweite Zacken (620) dazu konfiguriert sind, einen Abschnitt des Segels (120) zwischen sich zu halten.

13. Vorrichtung (100) gemäß Anspruch 12, darüber hinaus umfassend eine Hülle (610), die dazu konfiguriert ist, den ersten Zacken (615) gegen den zweiten Zacken (620) in der ersten Konfiguration zu drücken.

14. Vorrichtung (100) gemäß Anspruch 2, wobei der Segelanker (115) einen Kleber umfasst, vorzugsweise einen Kleber, der so formuliert ist, dass er sich nach einer bestimmten Zeitdauer auflöst.

15. Vorrichtung (100) gemäß einem der Ansprüche 1 bis 14, wobei der Primärführungsdraht (105) unabhängig von dem Sekundärführungsdraht (110) steuerbar ist.

## Revendications

1. Dispositif (100) destiné à faire avancer un fil-guide primaire (105) au-delà d'une valvule cardiaque, le dispositif (100) comprenant :
un fil-guide primaire (105) conçu pour avancer au-delà d'une valvule cardiaque ;
un fil-guide secondaire (110) conçu pour ouvrir en tirant un feuillet (120) de la valvule cardiaque tandis que le fil-guide primaire (105) avance au-delà de la valvule cardiaque ; et
un cathéter contenant à la fois le fil-guide primaire (105) et le fil-guide secondaire (110).

2. Dispositif (100) selon la revendication 1, dans lequel le fil-guide secondaire (110) comprend un ancrage de feuillet (115) conçu pour s'accoupler de manière amovible avec le feuillet (120) de la valvule cardiaque.

3. Dispositif (100) selon la revendication 2, dans lequel l'ancrage de feuillet (115) comprend une aiguille (425) conçue pour percer à travers une surface proximale du feuillet (120) et un crochet (420) conçu pour s'attacher à une surface distale du feuillet (120), la surface distale étant opposée à la surface proximale.

4. Dispositif (100) selon la revendication 2, dans lequel l'ancrage de feuillet (115) comprend une ventouse conçue pour s'accoupler avec une surface proximale du feuillet (120).

5. Dispositif (100) selon la revendication 2, dans lequel l'ancrage de feuillet (115) comprend une vis.

6. Dispositif (100) selon la revendication 2, dans lequel l'ancrage de feuillet (115) comprend une forme de cône ou une forme pyramidale présentant une extrémité pointue et une surface de base opposée à l'extrémité pointue, l'extrémité pointue étant conçue pour percer une surface proximale du feuillet (120) et la surface de base étant conçue pour venir en butée contre une surface distale du feuillet (120).

7. Dispositif (100) selon la revendication 2, dans lequel l'ancrage de feuillet (115) comprend une première dent (615) actionnable vers une première configuration et une seconde configuration et une seconde dent (620), dans lequel, dans la première configuration, la première dent (615) est sensiblement parallèle à la seconde dent (620) et, dans la seconde configuration, la première dent (615) est sensiblement perpendiculaire à la seconde dent (620).

8. Dispositif (100) selon la revendication 7 et comprenant en outre une gaine (610) conçue pour maintenir la première dent (615) dans la première configuration conjointement avec la seconde dent (620) en réponse au fait que la gaine (610) se trouve dans une première position.

9. Dispositif (100) selon la revendication 8, dans lequel la gaine (610) est conçue pour libérer la première dent (615) vers la seconde configuration en réponse au fait que la gaine (610) se trouve dans une seconde position, la seconde position étant atteinte par retrait de la gaine (610) à l'opposé d'une extrémité distale de l'ancrage de feuillet (115).

10. Dispositif (100) selon l'une quelconque des revendications 7 à 9, dans lequel la première dent (615) est conçue pour se courber vers un angle droit dans la seconde configuration.

11. Dispositif (100) selon la revendication 2, dans lequel l'ancrage de feuillet (115) comprend :
une première dent (615) actionnable vers une première configuration et une seconde configuration ;
une seconde dent (620) ; et
une articulation reliant la première dent (615) et la seconde dent (620) en un point éloigné des extrémités distales de la première dent (615) et de la seconde dent (620) ;
dans lequel, dans la première configuration, la première dent (615) presse contre la seconde dent (620) et dans la seconde configuration, la première dent (615) est séparée de la seconde dent (620).

12. Dispositif (100) selon la revendication 11, dans lequel, dans la première configuration, la première dent (615) et la seconde dent (620) sont conçues pour maintenir une partie du feuillet (120) entre elles.

13. Dispositif (100) selon la revendication 12 et comprenant en outre une gaine (610) conçue pour presser la première dent (615) contre la seconde dent (620) dans la première configuration.

14. Dispositif (100) selon la revendication 2, dans lequel l'ancrage de feuillet (115) comprend un adhésif, de préférence un adhésif qui est formulé pour se dissoudre après une certaine durée.

15. Dispositif (100) selon l'une quelconque des revendications 1 à 14, dans lequel le fil-guide primaire (105) peut être actionné indépendamment du fil-guide secondaire (110).
